# EUROPEAN PATENT APPLICATION

(11) **EP 0 974 364 A1**
(43) Date of publication of application: **26.01.2000**
(21) Application number: 97909616.1
(22) Date of filing: 23.10.1997
(51) Int. Cl.: A61K 47/14, A61K 47/24

(54) **METHOD OF INHIBITING DRUG PRECIPITATION**

(30) Priority: 25.10.1996 JP 28394996
(71) Applicant: YOSHITOMI PHARMACEUTICAL INDUSTRIES, LTD., Osaka-shi Osaka 541-0046 (JP)
(72) Inventor: KODAIRA, Hideto, Yoshitomo Pharm. Ind., Ltd., Osaka-shi, Osaka 541-0046 (JP); KIDO, Takae, Yoshitomo Pharmaceutical Indust.,Ltd., Osaka-shi, Osaka 541-0046 (JP); NAWA, Yoshihito, Yoshitomo Pharm. Ind., Ltd., Osaka-shi, Osaka 541-0046 (JP); MUNECHIKA, Koji, Yoshitomo Pharm. Ind., Ltd., Osaka-shi, Osaka 541-0046 (JP); UEDA, Yasuo, Yoshitomo Pharmaceutical Indust.,Ltd., Osaka-shi, Osaka 541-0046 (JP)
(74) Representative: VOSSIUS & PARTNER
(86) International application number: JP9703859
(87) International publication number: WO9818492

(57) **Abstract**

An acidic and/or a basic drug that may precipitate in aqueous solutions such as transfusion and the like, and at least one component from phospholipid, glyceride and synthetic surfactant are admixed.

Precipitation of a drug can be inhibited, that may have a decreased solubility due to changes in pH and form precipitates. Therefore, such drug can be administered under physiological pH conditions and by mixing the drug with a transfusion having the electrolyte composition suitable for the condition of patients, and administering same to the patients.

## Description

### Technical Field

The present invention relates to a method for inhibiting the precipitation of an acidic and/or a basic drug in aqueous solutions.

### Background Art

Thiopental and thiamylal are well known as general anesthetic and canrenoic add is well known as a diuretic to maintain urine amount during operation.

Thiopental and thiamylal are administered as a sodium salt thereof along with pre-operative transfusion and mid-operative transfusion (glucose-added electrolyte transfusion is often used) when introducing anesthesia, for the purpose of water supply and maintaining electrolytic balance. In addition, canrenoic acid is used as a potassium salt thereof upon admixture with the above-mentioned transfusions.

The injections containing these drugs contain the drug in the form of a salt dissolved in injectable water or physiological saline, wherein the pH of the aqueous solution after dissolution is about 9.0 - 11.5. The solubility of these drugs in water is pH-dependent, and when the pH of the aqueous solution becomes lower than 8.0 and approaches the neutral range, the solubility of the drug is known to become lower, whereby precipitates are produced. Accordingly, the directions attached to these drugs contain a statement calling attention to sufficient caution required in use for possible occurrence of precipitates due to changes in pH.

The above-mentioned transfusions to be used for the administration of these drugs generally have a pH of not more than 7.5 in consideration of stability of pharmaceutical preparation and the like. The label on these transfusions cautiously states, "Precipitate may occur upon admixture with thiopental sodium, thiamylal sodium, canrenoate potassium and the like. A solution that is not transparent after mixing should not be used." In clinical situations, therefore, these drugs are administered from a side tube of the drip tube under continuous attention paid to the generation of precipitate.

To avoid such problem, washing of the administration route, such as a drip tube and the like, with physiological saline after administration of these drugs has been proposed. When this method is used, however, an excess physiological saline is administered to patients, which may in turn misbalance the electrolyte.

In addition, the use of a transfusion having a high pH free of generation of precipitate or a transfusion having, alter admixture with a drug, a pH similar to the pH of the drug added and having low pH buffering capability has been tried, but such transfusions do not necessarily have the components suitable for the condition of patients.

### Disclosure of the Invention

With respect to the drug associated with the possibility of precipitation due to decreased solubility caused by changes in pH, the present inventors have conducted intensive studies in an attempt to inhibit precipitation and found that a certain component can inhibit precipitation of such drug and allows safe administration of the drug under physiological pH conditions, which resulted in the completion of the present invention.

Accordingly, the present invention provides a method for inhibiting precipitation of an acidic drug and/or a basic drug in aqueous solutions, which comprises mixing the drug(s) with at least one component from phospholipid, glyceride and synthetic surfactant (hereinafter also referred to as phospholipid etc.)

The drug to be the target of the inhibition of precipitation in the present invention is an acidic drug and/or a basic drug. In the present invention, these drugs are defined as follows. First, the acidic drug produces precipitates when the aqueous solution containing the drug has a pH lower than a specific pH, which is not more than 9.0, and decreases the solubility of the drug.

The basic drug produces precipitates when the aqueous solution containing the drug has a pH higher than a specific pH, which is not less than 5.0, and decreases the solubility of the drug.

The present invention can be applied in a broad range as long as the drug has the above-mentioned characteristics and is free of limitation on the kind thereof.

Specific examples include the following:
General anesthetic represented by thiopental and thiamylal (acidic drugs) and ketamine (basic drug),
Narco-anesthetic represented by amobarbital (acidic drug) and phenobarbital, midazolam and phenytrazepam (basic drug),
Antiepileptic, represented by phenytoin (acidic drug),
Antipyretic analgesic represented by ketoprofen (acidic drug) and buprenorphine and pentazocine (basic drugs),
Agent for neuro-psychosis represented by chlorpromazine, clomipramine, hydroxyzine and haloperidol (basic drugs),
Local anesthetic represented by lidocaine, procaine and bupivacaine (basic drugs),
Skeletal muscle relaxants represented by dantrolene (acidic drug) and vecuronium and papaverine (basic drugs),
Antispasmodic agent represented by atropine and scopolamine (basic drugs),
Antihistamic agent represented by chlorpheniramine (basic drug),
Cardiotonic represented by amrinone, aminophilline, dopamine and dobutamine (basic drugs),
Diuretic represented by canrenoic acid, furosemide and acetazolam (acidic drug),
Agent for arrhythmia represented by mexiletine and aprindine (basic drugs),
Hypotensive agent represented by reserpine and dihydroergotoxine (basic drugs),
Calcium antagonist represented by nicardipin (basic drug),
Vasodilator represented by dipyridamole and diltiazem (basic drugs),
Respiration promoter represented by doxapram (basic drug),
Protease inhibitor represented by nafamostat and gabexate (basic drugs),
Antitussive expectorant represented by bromhexine and ephedrine (basic drugs),
Adrenal hormone agent represented by hydrocortisone and methylprednisolone (acidic drugs),
Enzyme preparation represented by alteplase (acidic drug),
Antitumor agent represented by methotrexate (acidic drug) and nimustin, tegafur, doxorubicin, daunorubicin, aclarubicin, mitomycin C, dacarbazine, pirarubicin, epirubicin and mitoxantrone (basic drugs),
Antibiotics represented by ceftizoxime and cefodizime (acidic drug) and cefmenoxime and erythromycin (basic drugs),
Agents for chemotherapy represented by aciclovir, vidarabine, ganciclovir (basic drugs), and
Metoclopramide and cepharanthin (basic drugs) and the like.

The acidic drug may be in the form of an alkali metal salt such as sodium salt and the like, and the basic drug may be in the form of an inorganic acid salt such as hydrochloride and the like and organic acid salt such as mesilate and the like.

The present invention can be applied for the inhibition of the precipitation of a single drug, or simultaneous inhibition of the precipitation of plural drugs.

The drugs may be a combination of acidic drugs, basic drugs, or an acidic drug and a basic drug.

Particularly in the combination of an acidic drug and a basic drug, it is strikingly beneficial that the both can be administered simultaneously under the physiological pH conditions without precipitation.

Phospholipid is free of limitation as long as it is physiologically acceptable, metabolizable and nontoxic, and can be used in the present invention. For example, phosphatidylcholine, phosphatidylserine, phosphatidic acid, phosphatidylglycerine, phosphatidylethanolamine, phosphatidylinositol, phosphatidylglycerol, sphingomyelin and the like, mixtures thereof (e.g., soybean phospholipid, eggyolk phospholipid and complete or partially hydrogenated compounds thereof), and the like can be used.

Glyceride is phydiologically acceptable monoglyceride, diglyceride or triglyceride. Examples thereof include glycerol-1-acetate, glycerol-2-acetate, glycerol octadecyl ether, glycerol monooleate, glycerol monostearate, glycerol-1,3-diacetate, glycerol-1,3-dioleate, glycerol-1,2-distearate, glycerol-1,3-distearate, glycerol-1,2-dipalmitate, glycerol-1,3-dipalmitate, glycerol triacetate, glycerol tristearate, glycerol tripalmitate, glycerol tributylate, glycerol trimyristate, glycerol trilaurate, glycerol tris(2-ethylhexanoate), glycerol tri-12-hydroxyoctadecanoate, glycerol monostearate monocitrate, glycerol monostearate monotartrate, glycerol monostearate monolactate and the like. Preferred are medium chain fatty acid triglyceride [e.g., Panacet (trademark), ODO (trademark) and the like], chemically synthesized triglyceride [e.g., 2-linoleoyl-1,3-dioctanoylglycerol (8L8), 2-linoleoil-1,3-didecanoylglycerol (10L10) and the like].

The synthetic surfactant may be any as long as it is physiologically acceptable to be used in the present invention. Preferred are nonionic surfactant, which is exemplified by sodium N-cocoyl-N-methylaminoethylsulfonate, sucrose fatty add ester, squalane, steaxyl alcohol, polyoxyl 40 stearate, sorbitan sesquioleate, cetanol, cetomacrogol 1000, macrogol 400, polysorbate 60, polysorbate 80, polyoxyethylene hydrogenated castor oil 60, polyoxyethylene(105)polyoxypropylene(5)glycol, polyoxyethylene(120)polyoxypropylene(40)glycol, polyoxyethylene(160)polyoxypropylene(30)glycol, polyoxyethylene(20)polyoxypropylene(20)glycol, polyoxyethylenelauryl ether, glyceral monostearate, sorbitan monostearate, sodium laurylsulfate, lauromacrogol and the like.

### Detailed Description of the Invention

The inventive method for inhibiting precipitation can be realized in various manners. For example, the following modes are exemplified but the present invention is not limited to them.
① A mode wherein a drug and phospholipid etc. are previously contained in a composition and formulated into a preparation
   While the mode of addition is not particularly limited, for example, phospholipid etc. can be added to a drug in the form of a liposome or a fat emulsion. It may be formulated as a liposome preparation containing the drug or a fat emulsion preparation containing the drug. The methods for producing a liposome and a fat emulsion and the like are explained in the following.
   i) Preparation of liposome
      A phospholipid is dissolved in a solvent such as chloroform, ethanol and the like and thoroughly mixed. The inside of a container is dried under reduced pressure to evaporate the solvent. As a result, the phospholipid remain thin on the inside of the container, whereby a phospholipid film is formed. In this case, an antioxidant (e.g., tocopherol, vitamin E) is preferably added for the stabilization of phospholipid, preferably in a weight % relative to the phospholipid of about 0.01 - 0.5% (w/w).
      To this film of phospholipid are added physiological saline and the like and the mixture is vigorously shaken, preferably by ultrasonication, to homogeneously disperse the lipid, whereby a liposome is formed. The solvent in which the liposome is to be dispersed may be any as long as it does not degenerate or decompose the liposome and it is physiologically acceptable. Examples thereof include water (preferably injectable water, physiological saline and the like), ethanol and the like. The liposome prepared in this way is added to a drug.
      A liposome can be also prepared by a different method wherein a drug is added to a phospholipid solution in advance, the solvent is evaporated to form a phospholipid film having the drug attached thereto, a solvent is added and the mixture is subjected to centrifugation and the like to give a liposome, or other method. When preparing a film of phospholipid, cholesterol, phosphatidic acid, diacetyl phosphate and the like may be added as stabilizers.
      In addition, a high molecular weight substance selected from albumin, dextran, vinyl polymer, non-ionic surfactant, gelatin and hydroxyethyl starch may be contained as a stabilizer of liposome. The stabilizer of the high molecular weight substance may be taken into the void in the liposome, or added to a solution wherein liposome has been dispersed (namely, addition outside the liposome). It is naturally acceptable that the stabilizer be added inside and outside the liposome. The stabilizer is added in a proportion of 0.5 - 10 parts by weight, preferably 1 - 5 parts by weight, per part by weight of phospholipid.
   ii) Preparation of fat emulsion
      The fat and oil to be used for the fat emulsion are generally exemplified by vegetable oil, medium chain fatty acid triglyceride (MCT) and the like. The vegetable oil may be soybean oil, olive oil, safflower oil, corn oil, sesame oil, cottonseed oil, peanut oil, castor oil and the like, with preference given to soybean oil. The fat and oil are preferably added in a proportion of 1-50% (w/v), more preferably 2-30% (w/v), of a fat emulsion.
      The fat emulsion is prepared by a known production method, such as the following. That is, certain amounts of fat and oil (e.g., soybean oil), phospholipid etc. and other additive and the like are added as necessary, and heated where necessary to give a solution, which is treated to give a homogenous solution using a typical homogenizer (e.g., high pressure ejection type homogenizer, ultrasonic homogenizer and the like), whereby a water-in-oil type dispersion is given. Thereto is added a necessary amount of water and the mixture is homogenized using the above-mentioned homogenizer and converted to an oil-in-water type emulsion. For the convenient production, an additive such as stabilizer, isotonizing agent and the like may be added after generation of the fat emulsion (Japanese Patent Unexamined Publication Nos. 56-167161, 58-222014). A fat emulsion may be also prepared using a phospholipid etc. and an aqueous solution containing a drug. The fat emulsion preferably has an average particle size of not more than 0.3 µm, more preferably not more than 0.17 µm.
   iii) In addition to the above-mentioned mode, a drug and a phospholipid etc. are concealed in a container, such as a vial and the like, and the composition is freeze-dried under reduced pressure and dissolved in injectable water, physiological saline and the like when in use.
② A mode wherein an integrated kit for admixture when in use, which contains a drug and phospholipid etc., is used.
   For example, using a synthetic resin bag (plastic container) having plural compartments divided by a removable connection, a drug or an aqueous solution thereof is concealed in one of the compartments, and phospholipid etc. is concealed in a different compartment, and the connection is removed when in use to allow admixing of the components in the bag for use.
   In addition, an integrated kit containing a vial in which a drug is concealed, a synthetic resin bag (plastic bag) in which a phospholipid etc. are concealed and connecting needle to connect the both or syringe having plural compartments can be used suitably.
③ Besides the above, a mode wherein a drug and a phospholipid etc. are added when in use (combined type kit for preparation when in use), such as a combined type kit containing a drug and an attached solution for dissolution which contains a phospholipid etc., and a mode wherein a phospholipid etc. are previously added to transfusion and the like and a drug is mixed with the transfusion and the like for use are exemplified.
   The content of the phospholipid etc. is increased or decreased depending on the kind of the drug, the mode of lipid dispersion and the like. Therefore, there exists no suitable content common to all drugs. Generally, however, when a phospholipid is added in the form of a liposome, the liposome content is not less than 0.5 part by weight, preferably not less than 1 part by weight, more preferably not less than 2 parts by weight, per part by weight of the drug. When a phospholipid etc. is added in the form of a fat emulsion, the content of fat emulsion is not less than 0.05 part by weight, preferably not less than 0.1 part by weight, per part by weight of the drug.
The mode and the method for addition of the drug and phospholipid etc. are not particularly limited, as mentioned above. The presence of the phospholipid etc. can inhibit precipitation of the drug in, for example, a neutral solution. Therefore, such drug can be administered under the physiological pH conditions, and by mixing the drug with a transfusion having an electrolyte composition depending on the condition of patients and administering the same to the patients.

### Examples

The present invention is explained in more detail in the following by way of Reference Examples and Examples.

### Reference Example 1

Purified eggyolk lecitin (1.0 g) was dissolved in chloroform and the solvent was evaporated under reduced pressure in an eggplant flask. To this flask was added physiological saline to the total amount of 50 ml and the purified eggyolk lecitin was dispersed in the physiological saline by a Vortex mixer. This dispersion of purified eggyolk lecitin was ultrasonicated to give a homogeneous dispersion of purified eggyolk lecitin (liposome solution, 2 w/v%).

### Reference Example 2

Purified eggyolk lecitin (5.0 g) was dissolved in chloroform and treated in the same manner as in Reference Example 1 to give a homogeneous dispersion of purified eggyolk lecitin (liposome solution, 10 w/v%).

### Example 1

Thiamylal sodium (0.5 g, trademark : Isozol, manufactured by Yoshitomi Pharmaceutical Industries, Ltd.) was dissolved in injectable water (5 ml) and to this solution (0.5 ml) was added the liposome solution prepared in Reference Examples 1 and 2 by 1 ml and 5 ml, respectively. The obtained mixtures were added to McIlvaine's buffer solutions (pH 8.0 and pH 7.0) and appearance was observed. As the control, physiological saline (5 ml) was added instead of the liposome solution and used. The results are shown in Table 1.

**Table 1**

| | Liposome solution (2%) | | Liposome solution (10%) | | physiological saline |
|---|---|---|---|---|---|
| | 1 ml | 5 ml | 1 ml | 5 ml | |
| pH 7.0 | ± | - | - | - | ++ |
| pH 8.0 | - | - | - | - | - |

| | | | | | |
|---|---|---|---|---|---|
| - : Precipitation was not observed. | | | | | |
| ± : Slight precipitation was observed. | | | | | |
| + : Precipitation was observed. | | | | | |
| ++ : Noticeable precipitation was observed. | | | | | |

### Example 2

Sodium thiamylal (0.5 g, trademark : Isozol, manufactured by Yoshitomi Pharmaceutical Industries, Ltd.) was dissolved in injectable water (5 ml) and to this solution (0.5 ml) was added 20 w/v% soybean oil fat emulsion [trademark : 20 w/v% Intralipos (containing 1.2 w/v% purified eggyolk lecitin), manufactured by the Green Cross Corporation] or 20 w/v% MCT fat emulsion (containing 1.2 w/v% purified eggyolk lecitin) prepared using middle chain triglyceride (trademark : ODO, manufactured by THE NISSHIN OIL MILLS, LTD.) instead of soybean oil by 0.5 ml, 1.0 ml and 5.0 ml, respectively. The obtained mixtures were added to McIlvaine's buffer solutions (pH 5.0 - pH 8.0) and the appearance was observed. As the control, physiological saline was added instead of the fat emulsion and used. The results are shown in Table 2.

**Table 2**

| | soybean oil(20%) | | | MCT(20%) | | | physiological saline | | |
|---|---|---|---|---|---|---|---|---|---|
| | 0.5 ml | 1.0 ml | 5.0 ml | 0.5 ml | 1.0 ml | 5.0 ml | 0.5 ml | 1.0 ml | 5.0 ml |
| pH 5.0 | - | - | - | - | - | - | ++ | ++ | ++ |
| pH 6.0 | - | - | - | - | - | - | ++ | ++ | ++ |
| pH 7.0 | - | - | - | - | - | - | + | + | + |
| pH 8.0 | - | - | - | - | - | - | - | - | - |

| | | | | | | | | | |
|---|---|---|---|---|---|---|---|---|---|
| - : Precipitation was not observed. | | | | | | | | | |
| ± : Slight precipitation was observed. | | | | | | | | | |
| + : Precipitation was observed. | | | | | | | | | |
| ++ : Noticeable precipitation was observed. | | | | | | | | | |

### Example 3

Thiopental sodium (trademark : Ravonal, manufactured by TANABE SEIYAKU CO., LTD.) and potassium canrenoate (trademark : Soldactone, manufactured by DAINIPPON PHARMACEUTICAL CO. LTD.) were dissolved in the attached solution for dissolution. To each of the dissolved solutions (0.5 ml, respectively containing thiopental sodium 12.5 mg, potassium canrenoate 28.6 mg) was added 0.5 ml of a 20 w/v% soybean oil fat emulsion described in Example 2 or a 20 w/v% MCT fat emulsion described in Example 2. The obtained solutions were added to McIlvaine's buffer solutions (pH 5.0 - pH 8.0) and the appearance was observed. As the control, physiological saline (0.5 ml) was added instead of the liposome solution and used. The results are shown in Table 3.

**Table 3**

| | soybean oil(20%) | | MCT(20%) | | physiological saline | |
|---|---|---|---|---|---|---|
| | A | B | A | B | A | B |
| pH 5.0 | - | - | - | - | ++ | ++ |
| pH 6.0 | - | - | - | - | ++ | ++ |
| pH 7.0 | - | - | - | - | + | + |
| pH 8.0 | - | - | - | - | - | - |

| | | | | | | |
|---|---|---|---|---|---|---|
| A : thiopental sodium | | | | | | |
| B : potassium canrenoate | | | | | | |
| - : Precipitation was not observed. | | | | | | |
| ± : Slight precipitation was observed. | | | | | | |
| + : Precipitation was observed. | | | | | | |
| ++ : Noticeable precipitation was observed. | | | | | | |

### Example 4

Thiopental sodium (1 vial (300 mg), trademark : Ravonal, manufactured by TANABE SEIYAKU CO., LTD.), thiamylal sodium (1 vial (500 mg), trademark : Isozol, manufactured by Yoshitomi Pharmaceutical Industries, Ltd.) and potassium canrenoate (1 vial (200 mg),trademark : Soldactone, manufactured by DAINIPPON PHARMACEUTICAL CO. LTD.) were dissolved in 10 ml of a 20 w/v% soybean oil fat emulsion described in Example 2 or MCT fat emulsion described in Example 2. The obtained solutions were mixed with an electrolyte transfusion (trademark : Veen-D, 500 ml preparation, pH 5.4, manufactured by NIKKEN CHEMICALS CO., LTD.). Then, a drip tube was connected with the transfusion bag filled with each mixed solution and a filter (pore size 1.2 µm) was attached to the discharge end of the drip tube, and the content was discharged. As a control, each drug was dissolved in the attached solution for dissolution (12 ml for thiopental sodium, 20 ml for thiamylal sodium and 3.5 ml for potassium canrenoate) and mixed with the electrolyte transfusion and used. The results are shown in Table 4.

**Table 4**

| | soybean oil (20%) | MCT (20%) | Attached solution for dissolution |
|---|---|---|---|
| thiopental sodium | ○ | ○ | X |
| thiamylal sodium | ○ | ○ | X |
| potassium canrenoate | ○ | ○ | X |
| without addition | ○ | ○ | ○ |

| | | | |
|---|---|---|---|
| ○ : transfusable | | | |
| X : not transfusable | | | |

### Example 5

To amrinone (3 ml, trademark : cartonic injection,100 mg/20 ml, manufactured by YAMANOUCHI PHARMACEUTICAL CO., LTD.) was added a 10 w/v% soybean oil fat emulsion (trademark : 10 w/v% Intralipos, manufactured by the Green Cross Corporation) by 0.5 ml, 1.0 ml and 1.5 ml. To the obtained solutions was added McIlvaine's buffer solutions (5 ml, pH 5.0 - pH 8.0) and the mixture was centrifuged at 12000 rpm for 10 mm to confirm presence or otherwise of precipitation. As a control, physiological saline was added by 0.5, 1.0 and 1.5 ml instead of fat emulsion and used. The results are shown in Table 5.

**Table 5**

| | 10 w/v% soybean oil fat emulsion | | | physiological saline | | |
|---|---|---|---|---|---|---|
| | 0.5 ml | 1.0 ml | 1.5 ml | 0.5 ml | 1.0 ml | 1.5 ml |
| pH 5.0 | - | - | - | - | - | - |
| pH 6.0 | - | - | - | - | - | - |
| pH 7.0 | - | - | - | + | + | + |
| pH 8.0 | ± | - | - | ++ | ++ | ++ |

| | | | | | | |
|---|---|---|---|---|---|---|
| - : Precipitation was not observed. | | | | | | |
| ± : Slight precipitation was observed. | | | | | | |
| + : Precipitation was observed. | | | | | | |
| ++ : Noticeable precipitation was observed. | | | | | | |

### Example 6

To dipyridamole (0.3 ml, trademark : persantin injection, 10 mg/2 ml, manufactured by Boehringer Ingelheim) was added a 10 w/v% soybean oil fat emulsion described in Example 5 by 0.5 ml, 1.0 ml and 1.5 ml. To the obtained solutions was added McIlvaine's buffer solutions (5 ml, pH 5.0 - pH 8.0) and the mixture was centrifuged at 12000 rpm for 10 min to confirm presence or otherwise of precipitation. As a control, physiological saline was added by 0.5 ml, 1.0 ml and 1.5 ml instead of fat emulsion and used. The results are shown in Table 6.

**Table 6**

| | 10 w/v% soybean oil fat emulsion | | | physiological saline | | |
|---|---|---|---|---|---|---|
| | 0.5 ml | 1.0 ml | 1.5 ml | 0.5 ml | 1.0 ml | 1.5 ml |
| pH 5.0 | - | - | - | - | - | - |
| pH 6.0 | ± | - | - | + | + | + |
| pH 7.0 | ± | - | - | ++ | ++ | ++ |
| pH 8.0 | ± | - | - | ++ | ++ | ++ |

| | | | | | | |
|---|---|---|---|---|---|---|
| - : Precipitation was not observed. | | | | | | |
| ± : Slight precipitation was observed. | | | | | | |
| + : Precipitation was observed. | | | | | | |
| ++ : Noticeable precipitation was observed. | | | | | | |

### Example 7

Using a polyethylene sheet having a multi-layer structure, a plastic container consisting of 2 compartments divided by a connection capable of detaching was prepared. One of the compartments of the container was filled with erythromycin lactobionate (500 mg titer, trademark : injectable Exythrocin, manufactured by DAINIPPON PHARMACEUTICAL CO. LTD.). The 10 w/v% soybean oil fat emulsion (100 ml) described in Example 5 was dispensed to the other compartment. The connection between the two compartments was removed to mix erythromycin lactobionate and the fat emulsion. The mixed solution was mixed into an electrolyte transfusion (trademark : physiosol No. 3, 500 ml preparation, pH 5.0, manufactured by the Green Cross Corporation) from the side drip tube by a piggy bag method. A 0.8 µm filter was attached to the end of the drip tube, and the presence or otherwise of precipitation was detected. As a result, the entire amount of the mixed solution of erythromycin lactobionate and the fat emulsion passed trough the filter.

Using the above-mentioned container, a preparation containing physiological saline instead of the fat emulsion was prepared and the presence or otherwise of precipitation was detected. As a result, the 0.8 µm filter was clogged when 50 ml of erythromycin lactobionate dissolved in physiological saline was dripped.

### Example 8

Papaverine hydrochloride (40 mg, 1 ml, trademark : Papaverine hydrochloride injection, manufactured by DAINIPPON PHARMACEUTICAL CO. LTD.) was added to 10 w/v% soybean oil fat emulsion (10 ml) described in Example 5. This mixed solution was mixed into an electrolyte transfusion (trademark : Veen-F, 500 ml preparation, pH 7.0, manufactured by NIKKEN CHEMICALS CO., LTD.). A 0.8 µm filter was attached to the end of the drip tube, and the presence or otherwise of precipitation was detected. As a result, the entire amount of the mixed transfusion passed through the filter.

Separately, using the above-mentioned container, a preparation containing physiological saline instead of the fat emulsion was prepared and the presence or otherwise of precipitation was detected in the same manner. As a result, precipitation occurred when a physiological saline containing papaverine hydrochloride was mixed with the electrolyte transfusion and the 0.8 µm filter was clogged when 100 ml thereof was dripped.

### Example 9

Using a polyethylene sheet having a multi-layer structure, a plastic container consisting of 2 compartments divided by a connection capable of detaching was prepared. One of the compartments of the container was filled with a powder of ceftizoxime sodium (1 g titer, trademark : epocelin, manufactured by Fujisawa Pharmaceutical Co., Ltd.). The 10 w/v% soybean oil fat emulsion (100 ml) described in Example 5 was dispensed to the other compartment. The connection between the two compartments was removed to mix ceftizoxime sodium and the fat emulsion. The mixed solution was mixed into an electrolyte transfusion described in Example 8 from the side drip tube by a piggy bag method. A 0.8 µm filter was attached to the end of the drip tube, and the presence or otherwise of precipitation was detected. As a result, the entire amount of the mixed solution of ceftizoxime sodium and the fat emulsion passed through the filter.

Separately, using the above-mentioned container, a preparation containing physiological saline instead of the fat emulsion was prepared and the presence or otherwise of precipitation was detected in the same manner. As a result, the 0.8 µm filter was clogged when 80 ml thereof was dripped.

### Example 10

Using a polyethylene sheet having a multi-layer structure, a plastic container consisting of 2 compartments divided by a connection capable of detaching was prepared. One of the compartments of the container was filled with aciclovir (250 mg, trademark : Zovirax intravenous transfusion, manufactured by SUMITOMO PHARMACEUTICALS COMPANY LIMITED). The 10 w/v% soybean oil fat emulsion (100 ml) described in Example 5 was dispensed to the other compartment. The connection between the two compartments was removed to mix aciclovir and the fat emulsion. The mixed solution was mixed into an electrolyte transfusion described in Example 7 from the side drip tube by a piggy bag method. A 0.8 µm filter was attached to the end of the drip tube, and the presence or otherwise of precipitation was detected. As a result, the entire amount of the mixed solution of aciclovir and the fat emulsion passed through the filter.

Separately, using the above-mentioned container, a preparation containing physiological saline instead of the fat emulsion was prepared and the presence or otherwise of precipitation was detected in the same manner. As a result, the 0.8 µm filter was clogged when 40 ml of aciclovir dissolved in physiological saline was dripped.

### Example 11

Using a polyethylene sheet having a multi-layer structure, a plastic container consisting of 3 compartments divided by a connection capable of detaching was prepared. Each of the compartments of the container was filled with gabexate mesilate (100 mg, trademark : FOY for injection, manufactured by ONO PHARMACEUTICAL CO., LTD.), cefmenoxime hydrochloride (1 g, trademark : Bestcall for intravenous transfusion, manufactured by Takeda Chemical Industries, Ltd.) and 10 w/v% soybean oil fat emulsion (100 ml) described in Example 5. The respective connections between the compartments were removed to mix the contents. The mixed solution was dripped through a 0.8 µm filter, and the entire amount of the mixed solution could be dripped through the filter.

Separately, using the above-mentioned container, a preparation containing physiological saline instead of the fat emulsion was prepared and the presence or otherwise of precipitation was detected in the same manner. As a result, the 0.8 µm filter was clogged when about 30 ml of these drugs dissolved in physiological saline was dripped and the solution could not be dripped further.

### Example 12

Using a polyethylene sheet having a multi-layer structure, a plastic container consisting of 3 compartments divided by a connection capable of detaching was prepared. Each of the compartments of the container was filled with tegafur (400 mg, trademark : Futraful injection, manufactured by TAIHO PHARMACEUTICAL CO., LTD.), doxorubicin hydrochloride (20 mg titer, trademark : Adriacin injection, manufactured by KYOWA HAKKO KOGYO CO., LTD.) and 10 w/v% soybean oil fat emulsion (50 ml) described in Example 5. The connections between the compartments were removed to mix the contents. The mixed solution was dripped trough a 0.8 µm filter, and the entire amount of the mixed solution could be dripped through the filter.

Separately, using the above-mentioned container, a preparation containing physiological saline instead of the fat emulsion was prepared and the presence or otherwise of precipitation was detected in the same manner. As a result, the filter was clogged when about 40 ml of these drugs dissolved in physiological saline was dripped and the solution could not be dripped further. At this point, red precipitate was adhered to the filter.

### Example 13

Using a polyethylene sheet having a multi-layer structure, a plastic container consisting of 3 compartments divided by a connection capable of detaching was prepared. Each of the compartments of the container was filled with hydrocortisone (500 mg, trademark : Solu-Cortef, manufactured by SUMITOMO PHARMACEUTICALS COMPANY LIMITED), dipyridamole (10 mg, trademark : persantin injection,10 mg/2 ml, manufactured by Boehringer Ingelheim) and 10 w/v% soybean oil fat emulsion (50 ml) described in Example 5. The connections between the compartments were removed to mix the contents. The mixed solution was dripped through a 0.8 µm filter, and the entire amount of the mixed solution could be dripped through the filter.

Separately, using the above-mentioned container, a preparation containing physiological saline instead of the fat emulsion was prepared and the presence or otherwise of precipitation was detected in the same manner. As a result, these drugs dissolved in physiological saline precipitated and the filter was clogged when about 40 ml thereof was dripped and the solution could not be dripped further. At this point, yellow precipitate was adhered to the filter.

### Example 14

Using a polyethylene sheet having a multi-layer structure, a plastic container consisting of 3 compartments divided by a connection capable of detaching was prepared. Each of the compartments of the container was filled with methotrexate (200 mg, trademark : Methotrexate for injection, manufactured by Takeda Chemical Industries, Ltd.), doxorubicin hydrochloride (10 mg titer, trademark : Adriacin injection, manufactured by KYOWA HAKKO KOGYO CO., LTD.) and 10 w/v% soybean oil fat emulsion (50 ml) described in Example 5. The connections between the compartments were removed to mix the contents. The mixed solution was dripped through a 0.8 µm filter, and the entire amount of the mixed solution could be dripped through the filter.

Separately, using the above-mentioned container, a preparation containing physiological saline instead of the fat emulsion was prepared and the presence or otherwise of precipitation was detected in the same manner. As a result, these drugs dissolved in physiological saline precipitated and the filter was clogged when about 40 ml thereof was dripped and the solution could not be dripped further. At this point, red precipitate was adhered to the filter.

### Example 15

Using a polyethylene sheet having a multi-layer structure, a plastic container consisting of 3 compartments divided by a connection capable of detaching was prepared. Each of the compartments of the container was filled with cefodizime (500 mg, trademark : Kenicef, manufactured by TAIHO PHARMACEUTICAL CO., LTD.), gabexate mesilate (500 mg, trademark : FOY for injection 500, manufactured by ONO PHARMACEUTICAL CO., LTD.) and 10 w/v% soybean oil fat emulsion (50 ml) described in Example 5. The connections between the compartments were removed to mix the contents. The mixed solution was dripped through a 0.8 µm filter, and the entire amount of the mixed solution could be dripped through the filter.

Separately, using the above-mentioned container, a preparation containing physiological saline instead of the fat emulsion was prepared and the presence or otherwise of precipitation was detected in the same manner. As a result, these drugs dissolved in physiological saline precipitated and the filter was clogged when about 40 ml thereof was dripped and the solution could not be dripped further. At this point, yellow precipitate was adhered to the filter.

### Industrial Applicability

According to the present invention, a dug associated with the possibility of decreased solubility due to changes in pH and precipitation can be prevented from precipitation. Such drug can be administered under physiological pH conditions by mixing the drug with a transfusion having the electrolyte composition suitable for the condition of patients, and the like and administering same to the patients.

This application is based on application No. 283949/1996 filed in Japan, the content of which is incorporated hereinto by reference.

## Claims

1. A method for inhibiting precipitation of a drug in aqueous solutions, comprising mixing at least one component selected from the group consisting of phospholipid, glyceride and synthetic surfactant and an acidic drug and/or a basic drug.

2. The method of claim 1, wherein the acidic drug and/or the basic drug is a drug selected from the group consisting of general anesthetic, narco-anesthetic, antiepileptic, antipyretic analgesic, neuro-psychosis agent, local anesthetic, skeletal muscle relaxant, antispasmodic agent, antihistamic agent, cardiotonic, diuretic, agent for arrhythmia, hypotensive agent, calcium antagonist, vasodilator, respiration promoter, protease inhibitor, antitussive expectorant, adrenal hormone agent, enzyme preparation, antitumor agent, antibiotics and agent for chemotherapy.

3. The method of claim 1, wherein the acidic drug and/or the basic drug is a general anesthetic.

4. The method of claim 3, wherein the general anesthetic is at least one member selected from the group consisting of thiopental, thiamylal and a salt thereof.

5. The method of claim 1, wherein the acidic drug and/or the basic drug is a skeletal muscle relaxant.

6. The method of claim 5, wherein the skeletal muscle relaxant is papaverine.

7. The method of claim 1, wherein the acidic drug and/or the basic drug is a cardiotonic.

8. The method of claim 7, wherein the cardiotonic is amrinone.

9. The method of claim 1, wherein the acidic drug and/or the basic drug is a diuretic.

10. The method of claim 9, wherein the diuretic is canrenoic acid or a salt thereof.

11. The method of claim 1, wherein the acidic drug and/or the basic drug is a vasodilator.

12. The method of claim 11, wherein the vasodilator is dipyridamole.

13. The method of claim 1, wherein the acidic drug and/or the basic drug is a protease inhibitor.

14. The method of claim 13, wherein the protease inhibitor is gabexate or a salt thereof.

15. The method of claim 1, wherein the acidic drug and/or the basic drug is an adrenal hormone agent.

16. The method of claim 15, wherein the adrenal hormone agent is hydrocortisone or a salt threof.

17. The method of claim 1, wherein the acidic drug and/or the basic drug is an antitumor agent.

18. The method of claim 17, wherein the antitumor agent is at least one member selected from the group consisting of methotrexate, tegafur, doxorubicin, and a salt thereof.

19. The method of claim 1, wherein the acidic drug and/or the basic drug is an antibiotics.

20. The method of claim 19, wherein the antibiotics is at least one member selected from the group consisting of ceftizoxime, cefodizime, cefinenoxime, erythromycin and a salt thereof.

21. The method of claim 1, wherein the acidic drug and/or the basic drug is an agent for chemotherapy.

22. The method of claim 19, wherein the agent for chemotherapy is aciclovir or a salt thereof.

23. The method of claim 1, wherein the phospholipid is contained in the form of a liposome.

24. The method of claim 1, wherein at least one component selected from the group consisting of phospholipid, glyceride and synthetic surfactant is contained in the form of a fat emulsion.

25. A kit for preparation when in use comprising at least one component selected from the group consisting of phospholipid, glyceride and synthetic surfactant and an acidic drug and/or a basic drug in separation, the kit enabling inhibition of precipitation of the drug in aqueous solutions by mixing both when in use.
